# EUROPEAN PATENT APPLICATION

(11) **EP 1 160 716 A2**
(43) Date of publication of application: **05.12.2001**
(21) Application number: 01304761.8
(22) Date of filing: 31.05.2001
(51) Int. Cl.: G06F 19/00

(54) **System for automatically acquiring exam data from medical imaging devices and gererating reports on radiology department operations**

(30) Priority: 01.06.2000 US 208514 P; 27.10.2000 US 699167
(71) Applicant: GENERAL ELECTRIC COMPANY, Schenectady, NY 12345 (US)
(72) Inventor: Pomeroy, Bruce Douglas, Duanesburg, New York 12056 (US); White, Pauline, Delanson, New York 12053 (US); Butler, Timothy David, Waukesha, Wisconsin 53189 (US)
(74) Representative: Pedder, James Cuthbert

(57) **Abstract**

A method and apparatus for automatically providing reports on medical imaging devices (14, 16, 18). Specifically, a method and system for automatically obtaining examination process information from one or more medical imaging devices (14, 16, 18), automatically sending the data to an analysis center (12), and automatically providing useful reports to a customer.

## Description

The present invention relates generally to the field of medical diagnostic systems, such as imaging systems of various modalities. More particularly, the invention relates to a technique for providing service data and reports relating to the operative state of such diagnostic systems, service activities on the systems, historical performance data, and so forth.

This section is intended to introduce the reader to various aspects of art which may be related to various aspects of the present invention which are described and/or claimed below. This discussion is believed to be helpful in providing the reader with background information to facilitate a better understanding of the various aspects of the present invention. Accordingly, it should be understood that these statements are to be read in this light, and not as admissions of prior art.

Medical diagnostic and imaging systems are ubiquitous in modern health care facilities. Such systems provide invaluable tools for identifying, diagnosing and treating physical conditions and greatly reduce the need for surgical diagnostic intervention. In many instances, final diagnosis and treatment proceed only after an attending physician or radiologist has complemented conventional examinations with detailed images of relevant areas and tissues via one or more imaging modalities.

Currently, a number of modalities exist for medical diagnostic and imaging systems. These include computed tomography (CT) systems, x-ray systems (including both conventional and digital or digitized imaging systems), magnetic resonance (MR) systems, positron emission tomography (PET) systems, ultrasound systems, nuclear medicine systems, and so forth. In many instances, these modalities complement one another and offer the physician a range of techniques for imaging particular types of tissue, organs, physiological systems, and so forth. Health care institutions often dispose several such imaging systems at a single facility or at multiple facilities, permitting its physicians to draw upon such resources as required by particular patient needs.

Modern medical diagnostic systems typically include circuitry for acquiring image data and for transforming the data into a useable form, which is then processed to create a reconstructed image of features of interest within the patient. The image data acquisition and processing circuitry is often referred to as a "scanner" regardless of the modality, because some sort of physical or electronic scanning often occurs in the imaging process. The particular components of the system and related circuitry, of course, differ greatly between modalities due to their different physics and data processing requirements.

Medical diagnostic systems of the type described above are often called upon to produce reliable and understandable images within demanding schedules and over a considerable useful life. To ensure proper operation, the systems are serviced regularly by highly trained personnel who address imaging problems, configure and calibrate the systems, and perform periodic system checks and software updates. Moreover, service offerings have been supplemented in recent years by remote service centers capable of communicating with scanners at subscribing institutions. Such remote servicing is intended to maintain the diagnostic systems in good operational order without necessitating the attention of physicians or radiologists, and is often quite transparent to the institution.

In certain remote servicing systems, a computerized service center may contact a scanner via a network to check system configurations and operational states, to collect data for report generation, and to perform other useful service functions. Such contacts can be made periodically, such as during system "sweeps" in which a variety of system performance data is collected and stored with historical data for the particular scanner. The data can then be used to evaluate system performance, propose or schedule visits by service personnel, and the like.

While such service techniques have proven extremely valuable in maintaining diagnostic systems, further improvements are still needed. For example, in one type of conventional service system, contact between the scanners and a centralized service center most often originates with the service center. The scanners are provided with only limited functionality in the ability to identify and define service needs. Even where the scanners have permitted some limited ability to contact networked service providers, intermittent conditions indicative of a potentially serviceable problem may cease by the time the service provider is contacted or recontacts the scanner after a service call.

Other conventional systems provide some degree of interaction between service centers and institutions. In particular, an interactive service system is used to facilitate valuable exchanges of information, including reports of system performance, feedback on particular incidents requiring attention, updates of system licenses, software, imaging protocols, and so forth. However, these interactive systems which provide reports based on requests from medical personnel to the reporting center require a high degree of human intervention. Because of the intense need for human intervention, scanner data is generally provided to the reporting center in selected instances.

In addition to the foregoing drawbacks, conventional reporting systems are not controlled outside of the medical facility and may not be capable of processing meaningful reports based on comparison with other instruments, other labs and other hospitals. Further, conventional systems generally cannot provide reports and statistical models on within-exam procedures, since the diagnostic system data are often difficult to parse and do not contain metrics of such detail.

Radiology Administrators need greater visibility into their exam processes. In particular, they need metrics of system utilization and productivity. While this information can be extracted by human analysis, the labor involved is prohibitive, and an improved technique is needed for providing reports and other feedback on medical diagnostic and imaging systems and reporting data and metrics on system utilization and productivity. There is a particular need for a centralized reporting system which automatically provides periodic reports to subscribers based on current diagnostic and operational data and further provides historical data for comparative purposes. The system should provide comprehensive data which allows within-exam reporting as well as comparisons between other instruments.

The present invention may address one or more of the concerns set forth above.

Certain aspects commensurate in scope with the disclosed embodiments are set forth below. It should be understood that these aspects are presented merely to provide the reader with a brief summary of certain forms the invention might take and that these aspects are not intended to limit the scope of the invention. Indeed, the invention may encompass a variety of aspects that may not be set forth below.

In accordance with one embodiment of the present invention, there is provided a technique for reporting data to medical diagnostic systems and institutions designed to respond to these needs. The technique facilitates the automatic acquisition of information and the automatic preparation of reports. The reports may relate to service history for the system, the operative state of the system, periodic activities of various types performed by the system, financial performance of the system, and so forth. Certain reports may also include information relating to suggested or actual performance of the diagnostic system, such as suggested practices, system throughput, productivity solutions, system utilization, product support, training, operator performance, and the like.

The data and reports may also include or be based upon data relating to expanded populations of the same or similar types of diagnostic systems, permitting the operational state or history of particular systems to be compared. Where an institution includes a plurality of diagnostic systems, at a single or diverse locations, the reports may group the systems together in certain respects to facilitate comparative reporting. The specific reports requested and the format of the reports will be determined by the subscriber institutions. The reports may be automatically processed and automatically supplied to the user in several manners, such as by electronic delivery, postal delivery, FAX, or any other acceptable process. Other adaptations for requesting, compiling, transmitting, and accessing the reports are easily implemented by the present technique.

The invention will now be described in greater detail, by way of example, with reference to the drawings, in which:-
Fig. 1 illustrates a block diagram of an exemplary medical facility and servicing system in accordance with the present technique;
Fig. 2 illustrates an exemplary process flow in accordance with the present technique; and
Fig. 3 illustrates a block diagram of the base functions which take place at an analysis center.

One or more specific embodiments of the present invention will be described below. In an effort to provide a concise description of these embodiments, not all features of an actual implementation are described in the specification. It should be appreciated that in the development of any such actual implementation, as in any engineering or design project, numerous implementation-specific decisions must be made to achieve the developers' specific goals, such as compliance with system-related and business-related constraints, which may vary from one implementation to another. Moreover, it should be appreciated that such a development effort might be complex and time consuming, but would nevertheless be a routine undertaking of design, fabrication, and manufacture for those of ordinary skill having the benefit of this disclosure.

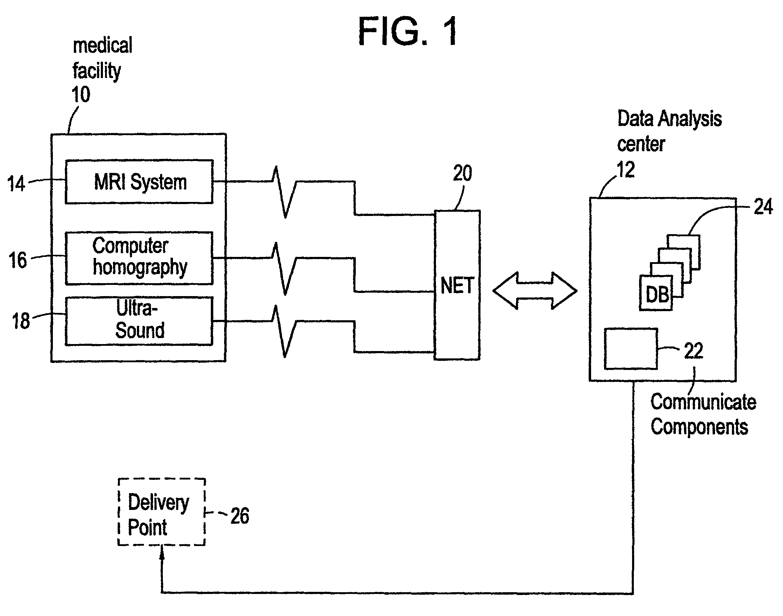

Turning now to the drawings, and referring initially to Fig. 1, an exemplary medical facility and servicing system is illustrated. This particular medical facility 10 includes a plurality of scanners which may be serviced by an analysis center 12. In one embodiment, the medical facility 10 includes an MRI system 14, a computed tomography system 16, and an ultrasound system 18. These and other modalities may be similarly serviced by the analysis center 12, depending upon the capabilities of the medical facility 10, the types of diagnostic systems subscribing to service contracts with the facility 10, as well as other factors. In general, the present technique is particularly well suited to providing remote service to a wide variety of medical diagnostic system modalities including, but not limited to, MRI systems, CT systems, ultrasound systems, positron emission tomography (PET) systems, nuclear medicine systems, and so forth. Service requests and data transmitted between the medical facility 10 and the analysis center 12 include data for identifying the type and modality of the system to be serviced. The modalities (e.g., MRI system 14, CT system 16, ultrasound system 18) may be linked to the analysis center 12 via a remote access network 20. For this purpose, any suitable network connection may be employed. Presently, advantageous network configurations may include both proprietary and dedicated networks as well as open networks, such as the Internet. Data may be exchanged between the various instruments in the medical facility 10 and the analysis center 12 in any suitable format, such as in accordance with Internet protocol, the transmission control protocol, or other known protocols. Moreover, certain of the data may be transmitted or formatted via markup languages such as hyper-text markup language (HTML), or the extensible markup language (XML), or other standard languages.

The analysis center 12 includes communication components 22, such as a network computer, which are configured to receive the communications from the medical facility 10. The communications components 22 are linked to one or more databases 24. The databases 24 may include information on operating parameters, service histories, and so forth, which are reserved for specific scanners and specific medical facilities 10 as well as external populations of diagnostic equipment. After receiving information from various scanners and processing the data, various reports are automatically produced by the analysis center 12. These reports are then sent to a designated delivery point 26. The delivery point 26 may be within the medical facility 10 or located remotely. The reports may be delivered by any specified means such as over the Internet, through e-mail, by fax, by mail, and so forth.

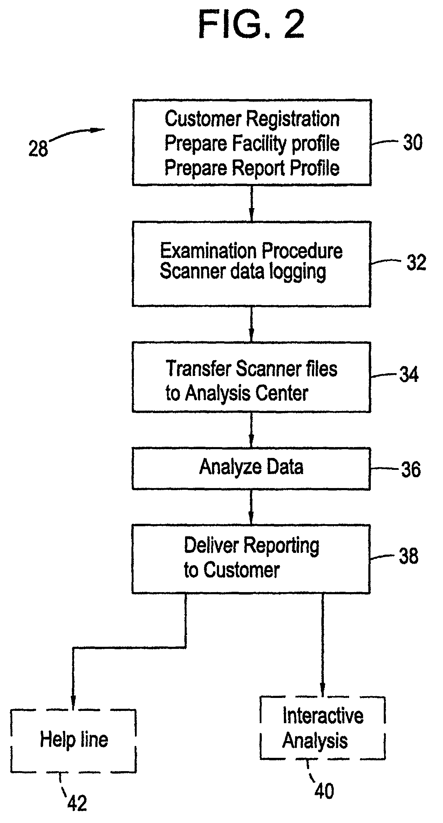

Fig. 2 illustrates a block diagram of an exemplary process flow incorporating the present technique as indicated generally at reference numeral 28. Initially, a customer, such as an administrator or other medical personnel from a medical facility, will register with an agent or representative of the analysis center, as in Block 30. Among the preliminary tasks associated with customer registration, the customer may prepare a Facility Profile which describes the scanners within the medical facility as well as the demographic, geographic and business characteristics of the facility. Further, the customer may complete a Report Profile which will be used by the analysis center to determine the type of reports and the format of the reports. The Report Profile may also indicate what type of peer comparison data that each medical facility desires. Once the profiles have been stored in the system at the analysis center, one or more databases are configured to store and maintain the data necessary for preparing each report. Data may be archived to create historical databases which may be used for peer comparisons through the reporting process. After the profiles are established, they are used to generate particular report formats for each subscriber. The reports will be generated using these formats which are stored in the database under each subscriber. Since report formats (which are based on the Report Profile) are stored in the database, the analysis center simply awaits the automatic receipt of the data from the medical facilities and automatically plugs the data and information into the current format assigned to each report. These profiles are initially delivered by the subscribing medical facility to the analysis center. Further, each scanner associated with a subscribing facility is linked to the analysis center by a network, such as a remote access network.

The examination procedure is generally indicated by block 32. During the examination procedure, a patient undergoes an imaging procedure, such as an MRI or CT procedure for instance. During an examination, both the MRI and CT operating software generates and saves files containing examination information such as imaging setup paramaters, examination codes, and date stamps to be used in preparing reports generated by the analysis center. Each scanner has a disk management strategy which is configured to retain data files until they are transferred to the analysis center.

Once the preliminary registration and linking is performed to establish a network link between a medical facility scanner and the analysis center, examination data logs are automatically transferred periodically to the analysis center as indicated at block 34. Exam logs are acquired from the digital imaging devices more frequently than previous techniques since the transfers are performed automatically and require no human intervention beyond the registration procedure, unless there are system failures. Thus, each scanner may be configured to deliver data log files twice each week, for instance. The frequency of this transfer may be selected or altered to avoid overflow of the scanner data storage media. The transfer software may be configured to establish expected arrival frequency for each customer scanner and send an alert to an appropriate personnel when a scanner appears to have dropped offline.

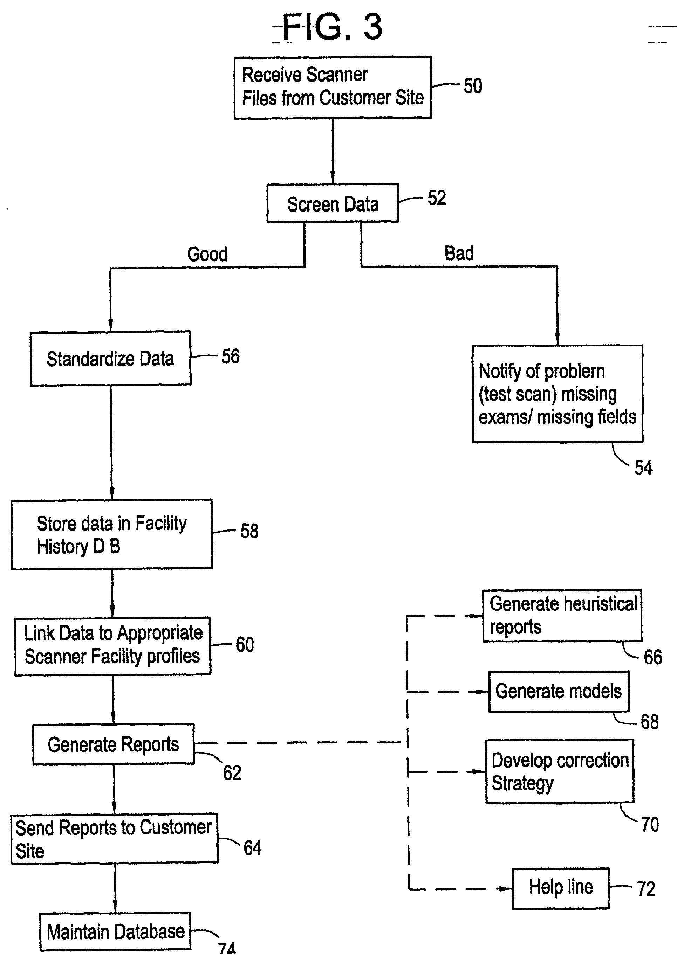

Next, the data received from the scanners is analyzed, as indicated by block 36 and further discussed with reference to Fig. 3. The analysis process facilitates appropriation of the customer report in accordance with the Report Profile established for each customer and computes the requested performance criteria such as volume, utilization, productivity, and so forth. A specific Report Profile may require that the examination data be analyzed and correlated with data from other scanners or other facilities. The results are displayed in a variety of formats including time series, pie charts and bar charts, for instance, as specified in each customer Report Profile.

Once the data is analyzed and the reports are prepared, the reports are delivered to the customer in accordance with a pre-established schedule, as indicated by block 38. The reports are delivered in a predetermined format specified in the Report Profile. The customer will receive reports as a mailed hard-copy, a fax, static pages that maybe viewed through an internet browser, or any other mutually acceptable format.

In addition to the automatic report system, the analysis center may also provide a second reporting technique such as an interactive analysis, as indicated by block 40. The interactive analysis system allows a subscriber to request user queries and allow near real-time interactive responses. Specific reports may be requested by the users. The databases at the analysis center may have precomputed information based on anticipated queries and may have certain reports and comparisons available immediately for those who request the information. For particularly unusual requests, response time for unique reports may be a few hours. However, the interactive analysis portion allows subscribers to request unique reports and information outside of their general Report Profile in response to unique needs.

Another optional portion of the analysis center may involve a help line, as indicated by block 42. The help line is essentially a operator based link between the medical facility and the analysis center. For instance, a hospital administrator may review the reports received from the analysis center but may have questions regarding the significance of certain data. The administrator can use the help line to speak with somebody directly at the analysis center who can provide them with specific insight regarding their reports. Further, the help line is available for immediate response to errors in system queries. Also, the help line may be used to begin advising on techniques to improve particular processes based on the reports. For instance, a medical facility administrator may notice that a particular scanner is being used inefficiently based on the reports. The help line can be used to begin consulting regarding how to improve the utilization or productivity of the particular scanner.

Fig. 3 illustrates the base functions which take place at the analysis center 12 (as illustrated in Fig. 1) during the data analysis (block 36 of Fig. 2). First, the analysis center automatically receives the scanner files from a subscribers medical facility, as in block 50. Again, once a subscription is made by the medical facility and a link is established to the analysis center, the scanner files will automatically be transmitted to the analysis center at a predetermined frequency. Once the data is received by the analysis center, the data is screened, as indicated by block 52. During the screening process, new scanner files are evaluated against several criteria. For instance, the files may be scanned for missing exams to determine if the file contains all of the exams that should have been acquired from each scanner in a period during this particular examination. This may be answered by comparing the exams seen in new files with those already in the facility history database. Also, a file may be screened to determine if there are missing fields critical to the reporting within a given file. It should be clear that other criteria may be used to screen the incoming data. If, after the screening process, missing or corrupted data are detected, an error flag may be sent to personnel at the analysis center and/or the data may be rejected. Service representatives at the analysis center can determine an appropriate course of action based on the type of failure. This notification process is indicated generally by block 54. If, on the other hand, all of the incoming data files pass the screening process, the data is standardized as indicated by block 56. Once the data is screened, the data must be standardized to categorize the exams in a manner that is significant to the recipients of the reports and consistent across all scanners in the database. Standardization is required for data fields that are typed by scanner operators; a simple example is patient sex which may be entered as M/F or male/female. A more complex example are exam descriptions and protocol names, which contain several words or abbreviations, and must be mapped to a standard study type classification such as the AMA Current Procedure Terminology (CPT). By standardizing the data received from different scanners and different medical facilities, reports may be prepared in such a way as to provide meaningful, quantitative comparisons between different examinations done on different scanners. In other words, a scanner file may contain abbreviations or synonymous terminology which should be categorized under a singular data type but which may otherwise end up being categorized under two or more separate data types. A database and software program may be provided to categorize all statements indicating a particular test result and assign them a single CPT code or name. One such program may provide a string-matching algorithm which compares segments of known terminology with segments of the current data. The algorithm will assign the most likely match from the database of known terminology. If there is no likely match, the algorithm may return an error message or a message indicating that no appropriate match was found and therefor, no appropriate match could be assigned. While a string-matching algorithm may be used, any standardization algorithm may be implemented to categorize like examination terminology and data..

Once the data is standardized, it is stored in the facility history database, as indicated by block 58. The database is used for preparing reports and possibly providing historical data for comparison to future scanner files. After storing a good data file in the facility history database, the data is linked to the appropriate scanner and facility profiles to provide the analysis center with the format and data necessary to prepare specified reports for a subscriber on a particular scanner or facility as indicated by block 60.

After the scanner data is standardized for meaningful comparison, stored in the facility database, and linked to the appropriate scanner profiles, reports can be generated based on Facility and Reporting Profiles, as previously discussed, indicated generally by block 62. The examination data may be parsed into groups or subgroups as required by the report profile for each customer and may compute requested performance indices, such as volume utilization, productivity, and so forth. The results may be displayed in a variety of formats including time series, pie charts, and bar charts as determined by the customer's Report Profile.

Next, these reports are automatically delivered to each customer's site or other location on a predetermined periodic basis, as indicated generally by block 64. Reports may be sent via the network link, the Internet, fax, U. S. mail, or any other mutually acceptable means of delivery. Regardless of the means of delivery, it should be noted that the reports are delivered on an automatic basis with no prompting from anyone associated with the medical facility after the initial subscription is made.

The analysis center may also provide certain other services as indicated generally by blocks 66, 68, 70, and 72. Aside from the automatic reporting, the analysis center may generate heuristical reports based on customer requests as indicated by block 66. The heuristical report generation is in an intelligent interpretation which reports statistics and detects trends and comparisons that are both practically and statistically significant. Heuristics may be used to select which features might be of interest to the customer based on his Facility Profile and Report Profile and additional text and graphics may be automatically generated in addition to the customer's report to display these particular observations. Further, models may be generated as indicated at block 68. Software tools within the analysis center may be used to survey the overall database for clusters of behavior and may be used to identify key variables and principle components to develop models which summarize the behavior within important clusters. These models may be used to establish standards for comparison of scanner and facility performance indices. The same models and data from the external sources may be used to assess bias and insufficient coverage of information within the database. Further, based on the assessments of imbalance in the database i.e., bias and sufficient coverage, personnel at the analysis center may be available to develop marketing strategies to expand the customer base in these areas to achieve or restore balance, or achieve statistical significance, within the reporting database. This development of a correction strategy is generally indicated at block 70. Also, as previously discussed, the analysis center may offer a help line, as indicated by block 72, to provide immediate assistance to medical facility personnel who have immediate questions regarding reports that they have received.

Also, included in the functions of the test analysis center is the maintenance of the database as indicated at block 74. Report format and databases must be continually managed. Incoming data must be distributed among relevant databases and archived for future comparative use. Further, the CPT codes and other standardized term sets database (as discussed with reference to block 60) must be continually updated with new and current terminology to assist in the standardization process.

The above-described base functions comprise an ordered listing of executable instructions for implementing logical functions. The ordered listing can be embodied in any computer-readable medium for use by or in connection with a computer-based system that can retrieve the instructions and execute them. In the context of this application, the computer-readable medium can be any means that can contain, store, communicate, propagate, transmit or transport the instructions. The computer readable medium can be an electronic, a magnetic, an optical, an electromagnetic, or an infrared system, apparatus, or device. An illustrative, but non-exhaustive list of computer-readable mediums can include an electrical connection (electronic) having one or more wires, a portable computer diskette (magnetic), a random access memory (RAM) (magnetic), a read-only memory (ROM) (magnetic), an erasable programmable read-only memory (EPROM or Flash memory) (magnetic), an optical fiber (optical), and a portable compact disc read-only memory (CD ROM) (optical). It is even possible to use paper or another suitable medium upon which the instructions are printed. For instance, the instructions can be electronically captured via optical scanning of the paper or other medium, then compiled, interpreted or otherwise processed in a suitable manner if necessary, and then stored in a computer memory.

For the sake of good order, various aspects of the invention are set out in the following clauses:-
1. A method of automatically providing reports on medical imaging devices comprising the acts of:
   (a) subscribing to receive reports on a plurality of medical imaging devices;
   (b) compiling a customized customer profile;
   (c) establishing a link between the medical imaging devices and a remote analysis center;
   (d) acquiring examination data using the medical imaging devices;
   (e) automatically transmitting the examination data to a remote analysis center;
   (f) automatically screening the examination data for errors;
   (g) automatically standardizing the examination data;
   (h) automatically storing the examination data in a centralized database;
   (i) automatically preparing reports based on the customized customer profile and the examination data; and
   (j) automatically delivering the reports to the customer.
2. The method of automatically providing reports on medical imaging devices, as set forth in clause 1, wherein act (a) comprises the act of providing a Facility Profile.
3. The method of automatically providing reports on medical imaging devices, as set forth in clause 1, wherein act (a) comprises the act of providing a Report Profile.
4. The method of automatically providing reports on medical imaging devices, as set forth in clause 1, wherein act (e) comprises the act of automatically transmitting the examination data to a remote analysis center at least twice each week.
5. The method of automatically providing reports on medical imaging devices, as set forth in clause 1, wherein act (f) comprises the act of screening the examination data for defects and missing data.
6. The method of automatically providing reports on medical imaging devices, as set forth in clause 1, wherein act (g) comprises the act of standardizing the examination data by assigning uniform exam terminology to like exam procedures.
7. The method of automatically providing reports on medical imaging devices, as set forth in clause 1, wherein act (g) comprises the act of assigning American Medical Association Current Protocol Terminology.
8. The method of automatically providing reports on medical imaging devices, as set forth in clause 1, wherein act (g) comprises the act of categorizing examination data using a string-matching algorithm.
9. The method of automatically providing reports on medical imaging devices, as set forth in clause 1, wherein act (h) comprises the act of interpreting the transmitted data and asserting the data into a database.
10. The method of automatically providing reports on medical imaging devices, as set forth in clause 1, wherein act (i) comprises the act of automatically preparing reports comprising comparative historical data.
11. The method of automatically providing reports on medical imaging devices, as set forth in clause 1, wherein act (i) comprises the act of automatically preparing reports comprising heuristical data.
12. The method of automatically providing reports on medical imaging devices, as set forth in clause 1, wherein act (i) comprises the act of automatically generating system models.
13. The method of automatically providing reports on medical imaging devices, as set forth in clause 1, wherein act (j) comprises the act of automatically delivering the reports to the customer via electronic delivery.
14. The method of automatically providing reports on medical imaging devices, as set forth in clause 1, further comprising the act of providing a user help line.
15. The method of automatically providing reports on medical imaging devices, as set forth in clause 1, further comprising the act of developing a correction strategy.
16. A method of automatically providing reports on medical imaging devices comprising the acts of:
   (a) automatically transmitting examination data acquired from the medical imaging devices to a remote analysis center;
   (b) automatically screening the examination data for errors;
   (c) automatically standardizing the examination data;
   (d) automatically storing the examination data in a centralized database;
   (e) automatically preparing reports based on a customized customer profile and the examination data; and
   (f) automatically delivering the reports to a customer.
17. The method of automatically providing reports on medical imaging devices, as set forth in clause 16, wherein act (a) comprises the act of automatically transmitting the examination data to a remote analysis center at least twice each week.
18. The method of automatically providing reports on medical imaging devices, as set forth in clause 16, wherein act (b) comprises the act of screening the examination data for defects and missing data.
19. The method of automatically providing reports on medical imaging devices, as set forth in clause 16, wherein act (c) comprises the act of standardizing the examination data by assigning uniform exam terminology to like exam procedures.
20. The method of automatically providing reports on medical imaging devices, as set forth in clause 16, wherein act (c) comprises the act of assigning American Medical Association Current Protocol Terminology.
21. The method of automatically providing reports on medical imaging devices, as set forth in clause 16, wherein act (c) comprises the act of categorizing examination data using a string-matching algorithm.
22. The method of automatically providing reports on medical imaging devices, as set forth in clause 16, wherein act (d) comprises the act of interpreting the transmitted data and asserting the data into a database.
23. The method of automatically providing reports on medical imaging devices, as set forth in clause 16, wherein act (e) comprises the act of automatically preparing reports comprising comparative historical data.
24. The method of automatically providing reports on medical imaging devices, as set forth in clause 16, wherein act (e) comprises the act of automatically preparing reports comprising heuristical data.
25. The method of automatically providing reports on medical imaging devices, as set forth in clause 16, wherein act (e) comprises the act of automatically generating system models.
26. The method of automatically providing reports on medical imaging devices, as set forth in clause 16, wherein act (f) comprises the act of automatically delivering the reports to the customer via electronic delivery.
27. A reporting network comprising:
   a medical facility comprising a plurality of medical imaging devices, the medical imaging devices configured to automatically transmit medical imaging data to a remote location; and
   a remote analysis center electronically coupled to the medical facility and configured to automatically receive the medical imaging data transmitted from the medical imaging devices and further configured to automatically generate reports from the data received from the medical imaging devices.
28. The reporting network, as set forth in clause 27, wherein the analysis center is electronically coupled to the medical facility via a computer network.
29. The reporting network, as set forth in clause 27, wherein the reports are automatically delivered to a predetermined location.
30. The reporting network, as set forth in clause 29, wherein the reports are automatically delivered to a customer via electronic delivery.
31. The reporting network, as set forth in clause 27, wherein the analysis center further comprises a historical database comprising medical imaging data taken from a plurality of medical imaging devices.
32. The reporting network, as set forth in clause 31, wherein the reports comprise data taken from the historical database.
33. A reporting network comprising a remote analysis center electronically coupled to a medical facility and configured to automatically receive medical imaging data transmitted from the medical imaging devices and further configured to automatically generate reports from the data received from the medical imaging devices.
34. The reporting network, as set forth in clause 33, wherein the analysis center is electronically coupled to a medical facility via a computer network.
35. The reporting network, as set forth in clause 33, wherein the reports are automatically delivered to a predetermined location.
36. The reporting network, as set forth in clause 35, wherein the reports are automatically delivered to a customer via electronic delivery.
37. The reporting network, as set forth in clause 33, wherein the analysis center further comprises a historical database comprising medical imaging data taken from a plurality of medical imaging devices.
38. The reporting network, as set forth in clause 37, wherein the reports comprise data taken from the historical database.
39. A reporting network comprising:
   a plurality of medical imaging devices configure to automatically transmit medical imaging data to a remote location; and
   a computer located at the remote location, the computer electronically coupled to the plurality of medical imaging devices and configured to automatically receive the medical imaging data transmitted from the data received from the medical imaging devices.
40. The reporting network, as set forth in clause 39, wherein the computer is electronically coupled to the medical facility via a computer network.
41. The reporting network, as set forth in clause 39, wherein the reports are automatically delivered to a predetermined location.
42. The reporting network, as set forth in clause 41, wherein the reports are automatically delivered to a customer via electronic delivery.
43. The reporting network, as set forth in clause 39, wherein the computer further comprises a historical database comprising medical imaging data taken from a plurality of medical imaging devices.
44. The reporting network, as set forth in clause 43, wherein the reports comprise data taken from the historical database.
45. A computer-readable medium storing computer instructions for:
   (a) automatically transmitting examination data acquired from medical imaging devices to a remote analysis center;
   (b) automatically screening the examination data for errors;
   (c) automatically standardizing the examination data;
   (d) automatically storing the examination data in a centralized database;
   (e) automatically preparing reports based on a customized customer profile and the examination data; and
   (f) automatically delivering the reports to a customer.
46. A tangible medium for storing a computer program comprising:
   (a) instructions for automatically transmitting examination data acquired from medical imaging devices to a remote analysis center;
   (b) instructions for automatically screening the examination data for errors;
   (c) instructions for automatically standardizing the examination data;
   (d) instructions for automatically storing the examination data in a centralized database;
   (e) instructions for automatically preparing reports based on a customized customer profile and the examination data; and
   (f) instructions for automatically delivering the reports to a customer.

## Claims

1. A method of automatically providing reports on medical imaging devices (14, 16, 18) comprising the acts of:
(a) subscribing to receive reports on a plurality of medical imaging devices;
(b) compiling a customized customer profile (30);
(c) establishing a link between the medical imaging devices (14, 16, 18) and a remote analysis center (12);
(d) acquiring examination data (32) using the medical imaging devices (14, 16, 18);
(e) automatically transmitting the examination data (34) to a remote analysis center (12);
(f) automatically screening the examination data (52) for errors;
(g) automatically standardizing the examination data (56);
(h) automatically storing the examination data (58) in a centralized database (24);
(i) automatically preparing reports (62) based on the customized customer profile and the examination data; and
(j) automatically delivering the reports (64) to the customer.

2. The method of automatically providing reports on medical imaging devices (14, 16, 18), as set forth in claim 1, wherein act (a) comprises the act of providing a Facility Profile.

3. A method of automatically providing reports on medical imaging devices (14, 16, 18) comprising the acts of:
(a) automatically transmitting examination data (34) acquired from the medical imaging devices (14, 16, 18) to a remote analysis center (12);
(b) automatically screening the examination data (52) for errors;
(c) automatically standardizing the examination data (56);
(d) automatically storing the examination data (58) in a centralized database (24);
(e) automatically preparing reports (62) based on a customized customer profile and the examination data; and
(f) automatically delivering the reports (64) to a customer.

4. The method of automatically providing reports on medical imaging devices (14, 16, 18), as set forth in claim 3, wherein act (a) comprises the act of automatically transmitting the examination data (34) to a remote analysis center (12) at least twice each week.

5. A reporting network comprising:
a medical facility (10) comprising a plurality of medical imaging devices (14, 16, 18), the medical imaging devices (14, 16, 18) configured to automatically transmit medical imaging data to a remote location; and
a remote analysis center (12) electronically coupled to the medical facility (10) and configured to automatically receive the medical imaging data transmitted from the medical imaging devices (14, 16, 18) and further configured to automatically generate reports from the data received from the medical imaging devices (14, 16, 18).

6. The reporting network, as set forth in claim 5, wherein the analysis center (12) is electronically coupled to the medical facility (10) via a computer network.

7. A reporting network comprising a remote analysis center (12) electronically coupled to the medical facility (10) and configured to automatically receive the medical imaging data transmitted from the medical imaging devices (14, 16, 18) and further configured to automatically generate reports from the data received from the medical imaging devices (14, 16, 18).

8. A reporting network comprising:
a plurality of medical imaging devices (14, 16, 18) configure to automatically transmit medical imaging data to a remote location; and
a computer located at the remote location, the computer electronically coupled to the plurality of medical imaging devices (14, 16, 18) and configured to automatically receive the medical imaging data transmitted from the data received from the medical imaging devices (14, 16, 18).

9. A computer-readable medium storing computer instructions for:
(a) automatically transmitting examination data (34) acquired from medical imaging devices (14, 16, 18) to a remote analysis center (12);
(b) automatically screening the examination data (52) for errors;
(c) automatically standardizing the examination data (56);
(d) automatically storing the examination data (58) in a centralized database (24);
(e) automatically preparing reports (62) based on a customized customer profile and the examination data; and
(f) automatically delivering the reports (64) to a customer.

10. A tangible medium for storing a computer program comprising:
(a) instructions for automatically transmitting examination data (34) acquired from medical imaging devices (14, 16, 18) to a remote analysis center (12);
(b) instructions for automatically screening the examination data (52) for errors;
(c) instructions for automatically standardizing the examination data (56);
(d) instructions for automatically storing the examination data (58) in a centralized database (24);
(e) instructions for automatically preparing reports (62) based on a customized customer profile and the examination data; and
(f) instructions for automatically delivering the reports (64) to a customer.
